# EUROPEAN PATENT SPECIFICATION

(11) **EP 2 144 583 B1**
(45) Date of publication and mention of the grant of the patent: **30.05.2012**
(21) Application number: 08719678.8
(22) Date of filing: 13.03.2008
(51) Int. Cl.: A61F 13/15

(54) **ARRAY OF GENDER-SPECIFIC, DISPOSABLE, ADULT-INCONTINENCE ARTICLES**
ANORDNUNG VON GESCHLECHTSSPEZIFISCHEN EINWEGARTIKELN FÜR INKONTINENZ BEI ERWACHSENEN
ENSEMBLE D' ARTICLES JETABLES ET SPÉCIFIQUES AU SEXE POUR INCONTINENTS ADULTES

(30) Priority: 04.05.2007 US 800343
(43) Date of publication of application: 20.01.2010
(73) Proprietor: KIMBERLY-CLARK WORLDWIDE, INC., Neenah, WI 54956 (US)
(72) Inventor: WHEELER, Katherine, C., Menasha, Wisconsin 54952 (US); FELL, David, A., Neenah, Wisconsin 54956 (US); RICHLEN, Sandra, A., Black Creek, Wisconsin 54106 (US); SIMON, Amanda, J., Oshkosh, Wisconsin 54901 (US); THOMAS, Brian, L., Appleton, Wisconsin 54915 (US)
(74) Representative: Davies, Christopher Robert
(86) International application number: PCT/IB2008/050925
(87) International publication number: WO 2008/135870

(56) References cited:
- WO-A-89/00037
- WO-A-2007/008125
- DE-U1- 29 814 465
- US-A1- 2003 093 045
- US-A1- 2003 130 643
- US-A1- 2004 143 231

## Description

People rely on disposable absorbent products In their everyday lives, Many manufacturers seek to better meet the needs of users of such products. Some manufacturers, for example, have sought to develop products that absorb better. Others have sought to improve fit.

Men and women have different body compositions (e.g., percentage of body mass consisting of muscle), different skeletal structures, and age differently. As men get older they tend to lose muscle mass in their legs, resulting in thinner legs. In fact, the circumference of the leg of a typical male user of an adult-incontinence article may actually be smaller than the circumference of the leg of a typical female user of an adult-incontinence article a counterintuitive finding. Also, for anatomical reasons, men typically release urine at a location further toward the front of the body, while women release urine at a location generally lower on the body compared to men. Furthermore, the shape of a man's hips and groin area is different from a woman's hips and groin area.

We are not aware of product designs, product lines, or ways of marketing that address these and other differing body characteristics of men and women who use disposable adult-incontinence products.

Prior art absorbent articles are disclosed in US2004/0143,231, WO-89/00037, US 2003/0093045 and DE29,814,465.

### Summary

We have conceived disposable absorbent product designs tailored to the differing anatomical features of a man's body and a woman's body. Furthermore, we have studied and taken into account the differing ways in which men and women age and typically distribute their body weight. Accordingly, we have conceived products, product lines, and ways of drawing attention to these products that address the aforementioned anatomical features, including those correlating with aging.

According to the present invention, there is provided an array as claimed in claim 1.

One representative version of the invention is an array of disposable adult-incontinence articles, the array comprising: a first disposable adult-incontinence article having a first anterior length, wherein the first article is adapted to be worn by women; and a second disposable adult-incontinence article having a second anterior length, wherein the second article is adapted to be worn by men, and wherein the first anterior length is less than the second anterior length. For purposes of this application, the term "anterior length" refers to the shortest distance between the waist-opening front edge and the leg-opening front edge.

Another representative version of the invention is an array of disposable incontinence articles, the array comprising: a first disposable adult-incontinence article having a first leg-opening front edge, wherein the first article is adapted to be worn by women; and a second disposable adult-incontinence article having a second leg-opening front edge, wherein the second article is adapted to be worn by men, and wherein the first leg-openlng front edge is more convex toward the waist-opening front edge than the second leg-opening front edge. That is, the boundary defined by the first leg-opening front edge of the article adapted to be worn by women curves more toward the waist-opening front edge (and away from the open space bounded or partially bounded by the leg-opening edges) compared to the boundary defined by the second leg-opening front edge of the article adapted to be worn by men. For purposes of this application, a women's article having this feature comprises a leg-opening front edge having (or, alternatively, establishing a boundary having) a greater convexity compared to the leg-opening front edge of the corresponding men's article.

Another representative version of the invention is an array of disposable incontinence articles, the array comprising: a first disposable adult-incontinence article having a first leg-opening back edge, wherein the first article is adapted to be worn by women; and a second disposable adult-incontinence article having a second leg-opening back edge, wherein the second article is adapted to be worn by men, and wherein the first leg-opening back edge is more concave toward the waist-opening back edge than the second leg-opening back edge. That is, the boundary defined by the first leg-opening back edge of the article adapted to be worn by women curves more away from the waist-opening back edge (and toward the open space bounded or partially bounded by the leg-opening edges) compared to the boundary defined by the second leg-opening back edge of the article adapted to be worn by men. For purposes of this application, a women's article having this feature comprises a leg-opening back edge having (or, alternatively, establishing a boundary having) a greater concavity compared to the leg-opening back edge of the corresponding men's article.

Another representative version of the invention is an array of disposable adult-incontinence articles, the array comprising: a first disposable adult-incontinence article having a first crotch width, wherein the first article is adapted to be worn by women; and a second disposable adult-incontinence article having a second crotch width, wherein the second article is adapted to be worn by men; and wherein the first crotch width is less than the second crotch width. For purposes of this application, the term "crotch width" refers to the shortest distance between the outside boundaries of the opposing, elastic members closest to the opposing leg-opening side edges. For example, if three elastic strands are employed near each leg-opening side edge, then "crotch width" equates to the shortest distance between: (1) of the three elastic strands proximate to a first leg-opening side edge, the outside boundary of that strand closest to the first leg-opening side edge; and (2) of the three elastic strands proximate to the opposing, second leg-opening side edge, the outside boundary of that strand closest to the opposing, second leg-opening side edge.

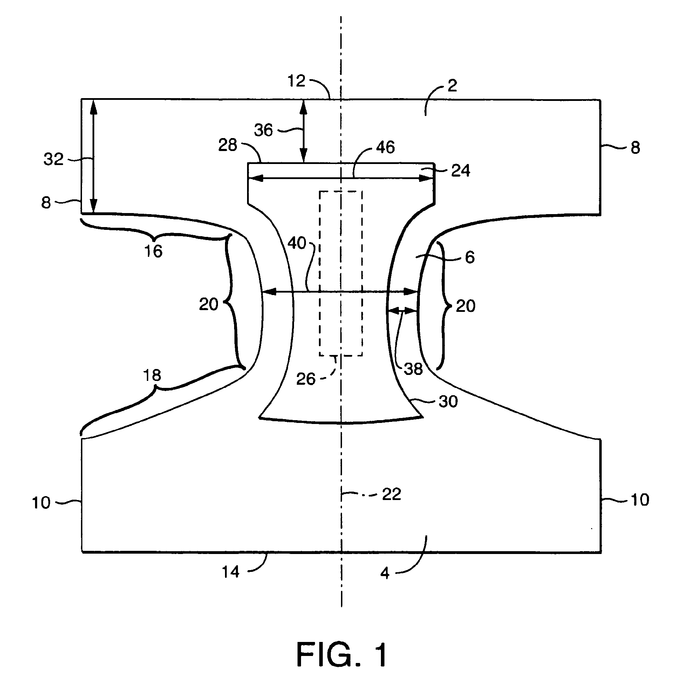

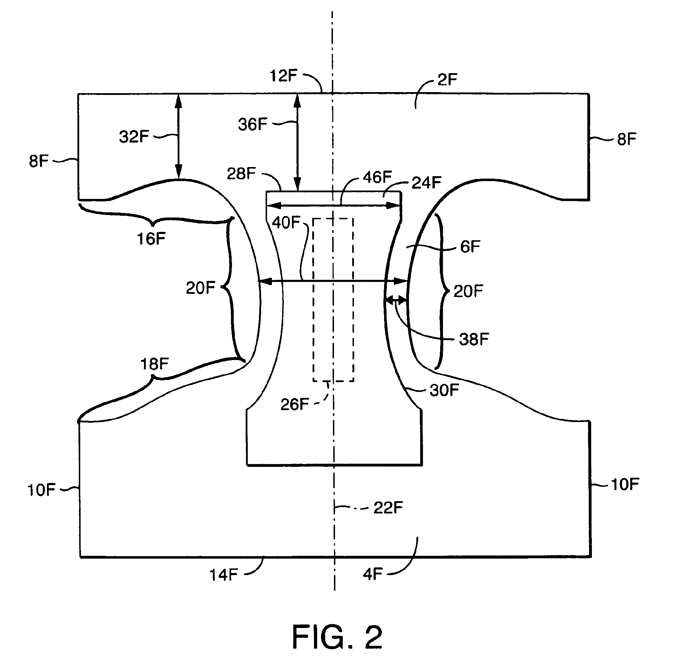

Another representative version of the invention is an array of disposable adult-incontinence articles, the array comprising: a first disposable adult-incontinence article having a first gasket width, wherein the first article is adapted to be worn by women; and a second disposable adult-incontinence article having a second gasket width, wherein the second article is adapted to be worn by men; and wherein the first gasket width is less than the second gasket width. For purposes of this application, the term "gasket width" refers to, in the crotch portion, the distance, along a line bisecting the middle of the leg-opening side edges in a transverse direction parallel to the waist-opening edges (see Figs. 1 and 2 and accompanying text), between a side edge of the absorbent core (e.g., a fluff / superabsorbent core) and the outside boundary of the elastic member closest to the nearest, leg-opening side edge If, as is likely, the absorbent core, such as a core comprising fluff and superabsorbent, is disposed between a liquid-permeable, body-facing liner and a liquid-impermeable backsheet (as with an absorbent insert or assembly comprising an absorbent core sandwiched between a liner and backsheet), then the side edge of the absorbent core is located at the boundary of the fluff / superabsorbent core, not at the boundary of any substrate that helps encase and extends beyond the absorbent core.

Another representative version of the invention is an array of disposable adult-incontinence articles, the array comprising: a first disposable adult-incontinence article having a first leg-opening perimeter, wherein the first article is adapted to be worn by women; and a second disposable adult-Incontinence article having a second leg-opening perimeter, wherein the second article is adapted to be worn by men; and wherein the first leg-opening perimeter is greater than the second leg-opening perimeter. For purposes of this application, the term "leg-opening perimeter" refers to the perimeter of a leg opening in a substantially flat, laid-open, disposable adult-incontinence article. The exemplary articles displayed in Figures 1 and 2 are in a substantially flat, laid-open condition.

As noted generally above, the present invention involves various combinations of the aforementioned characteristics Including one, some, or all of the aforementioned characteristics.

Another representative version of the invention is an array of disposable adult-incontinence articles comprising one or more of the gender-specific features discussed above, with the first disposable adult-incontinence article further comprising an absorbent having a first waist/absorbent distance, wherein the first article is adapted to be worn by women; and a second disposable adult-incontinence article having a second waist/absorbent distance, wherein the second article is adapted to be worn by men; and wherein the first waist/absorbent distance is more than the second waist/absorbent distance. The term "waist/absorbent distance" refers to the minimum distance between the front edge of the waist opening and front edge of the absorbent core. Again, as discussed above, the edge of the absorbent core refers to, for example, the edge of a fluff / superabsorbent core (not the edge of substrates that help contain the core if, as may be the case, the edge of these substrates extend beyond the boundary of the absorbent core itself).

Another representative version of the invention is an array of disposable adult-incontinence articles comprising one or more of the dimensional (e.g., those inventive features directed to a length, width, distance, or other such measurement) or ornamental (e.g., those inventive features directed to disposing a graphic on an article) gender-specific features discussed above, with the first disposable adult-incontinence article further comprising an absorbent core having a first frontal area, wherein the first article is adapted to be worn by women; and a second disposable adult-incontinence article having an absorbent core having a second frontal area, wherein the second article is adapted to be worn by men; and wherein the first frontal area is less than the second frontal area. The term "frontal area," when describing the absorbent core (e.g., a fluff / superabsorbent core), refers to the area of the body-facing surface of the absorbent core forward of an imaginary transverse line bisecting the article, with area determined when the article comprising the absorbent core is in a substantially flat, laid-open position (e.g., for some articles, the substantially flat, laid-open position generally corresponds to an hourglass-like shape). For example, if an article is in a substantially flat, laid-open position, and has an hourglass-like shape like those versions depicted in Figs. 1 and 2 (and which have a shape generally analogous to the capital letter "I"), an imaginary transverse line would be a horizontal line halfway between the waist-opening front edge and the waist-opening back edge (i.e., the imaginary transverse line would be a horizontal line half-way between the upper and lower horizontal lines that make up the letter "I").

Another representative version of the invention is an array of disposable adult-incontinence articles comprising one or more of the dimensional (e.g., those inventive features directed to a length, width, distance, or other such measurement) or ornamental (e.g., those inventive features directed to disposing a graphic on an article) gender-specific features discussed above, with the first disposable adult-incontinence article further comprising an absorbent core having a first frontal transverse span, wherein the first article is adapted to be worn by women; and a second disposable adult-incontinence article having an absorbent core having a second frontal transverse span, wherein the second article is adapted to be worn by men; and wherein the first frontal transverse span is less than the second frontal transverse span. The term "frontal transverse span," when describing the absorbent core (e.g., a fluff / superabsorbent core), refers to the longest distance, in a transverse direction parallel to the waist-opening edges (see Figs. 1 and 2 and accompanying text), between the opposing, longitudinal edges of the absorbent core forward of an imaginary transverse line bisecting the article In a substantially flat, laid-open position (e.g., for many articles, the substantially flat, laid-open position generally corresponds to a hourglass-like shape).

A disposable, adult-incontinence article may employ a pair of containment flaps that are configured to provide a barrier to the transverse flow of body exudates. A flap elastic member can be operatively joined with each containment flap in any suitable manner as is well known in the art. The elasticized containment flaps define an unattached edge which assumes an upright, generally perpendicular configuration to form a seal against the wearer's body. The opposing end of the flap is attached to the article (e.g., at a position inward of the leg-opening side edges). Suitable constructions and arrangements for the containment flaps are generally well known to those skilled in the art and are described, for example, in U.S. Pat. No. 4,704,116 issued Nov. 3, 1987 to Enloe. Accordingly, another representative version of the Invention is an array of disposable adult-incontinence articles comprising one or more of the dimensional (e.g., those inventive features directed to a length, width, distance, or other such measurement) gender-specific features discussed above, with one or both gender-specific articles employing containment flaps.

Also disclosed is an array of disposable adult-incontinence articles comprising one or more of the inventive dimensional (e.g., those inventive features directed to a length, width, distance, or other such measurement), ornamental (e.g., those features directed to disposing a graphic on an article) or other gender-specific features discussed above, with the first disposable adult-incontinence article contained in a first package having a first statement disposed thereon, wherein the first article is adapted to be worn by women; and a second disposable adult-incontinence article contained in a second package having a second statement disposed thereon, wherein the second article is adapted to be worn by men; wherein the first statement associates the article with use by women and the second statement associates the article with use by men.

For purposes of this application, "fit features" refer to one or more of anterior length, shape of leg-opening front edge (i.e., degree of convexity of said edge), shape of leg-opening back edge (i.e., degree of concavity of said edge), leg-opening perimeter, and gasket width. Also, for purposes of this application, "leg-opening features" refers to one or more of anterior length, shape of leg-opening front edge, shape of leg-opening back edge, and leg-opening perimeter.

Also disclosed is a disposable, adult-incontinence article comprising a fit feature adapted for a specific gender, or various combinations of those features denominated as fit features, and which are adapted for a specific gender.

Also disclosed is a disposable, adult-incontinence article comprising a leg-opening feature adapted for a specific gender or various combinations of those features denominated as leg-opening features, and which are adapted for a specific gender.

The various representative articles described in the preceding two paragraphs may, of course, be contained in packages on which are disposed statements associating the articles contained therein with use by a specific gender. Also disclosed is a method of providing a gender-specific, disposable, adult-incontinence article, the method comprising the steps of transmitting a statement embodied in a tangible medium that refers to a gender-specific, disposable, adult-incontinence article comprising a fit feature and/or a leg-opening feature; and positioning in the hands of a user a disposable, adult-incontinence article comprising a fit feature and/or a leg-opening feature, wherein the article adapted to fit the gender of the user.

### Drawings

Figure 1 depicts one representative example of a disposable adult-incontinence article adapted to be worn by a man.
Figure 2 depicts one representative example of a disposable adult-incontinence article adapted to be worn by a woman.
Figure 3 depicts one representative example of a disposable adult-incontinence article adapted to be worn by a man.
Figure 4 depicts one representative example of a disposable adult-incontinence article adapted to be worn by a woman.

Within the context of this specification, each term or phrase below includes the following meaning or meanings:
"Attach" and its derivatives refer to the joining, adhering, connecting, bonding, sewing together, or the like, of two elements. Two elements will be considered to be attached together when they are integral with one another or attached directly to one another or indirectly to one another, such as when each is directly attached to intermediate elements. "Attach" and its derivatives include permanent, releasable, or refastenable attachment. In addition, the attachment can be completed either during the manufacturing process or by the end user.
"Bond" and its derivatives refer to the joining, adhering, connecting, attaching, sewing together, or the like, of two elements. Two elements will be considered to be bonded together when they are bonded directly to one another or indirectly to one another, such as when each is directly bonded to intermediate elements. "Bond" and its derivatives include permanent, releasable, or refastenable bonding.
"Coform" refers to a blend of meltblown fibers and absorbent fibers such as cellulosic fibers that can be formed by air forming a meltblown polymer material while simultaneously blowing air-suspended fibers into the stream of meltblown fibers. The coform material may also include other materials, such as superabsorbent materials. The meltblown fibers and absorbent fibers are collected on a forming surface, such as provided by a foraminous belt. The forming surface may include a gas-pervious material that has been placed onto the forming surface.
"Connect" and its derivatives refer to the joining, adhering, bonding, attaching, sewing together, or the like, of two elements. Two elements will be considered to be connected together when they are connected directly to one another or indirectly to one another, such as when each is directly connected to intermediate elements. "Connect" and its derivatives include permanent, releasable, or refastenable connection. In addition, the connecting can be completed either during the manufacturing process or by the end user.
"Disposable" refers to articles which are designed to be discarded after a limited use rather than being laundered or otherwise restored for reuse.
The terms "disposed on," "disposed along," "disposed with," or "disposed toward" and variations thereof are intended to mean that one element can be integral with another element, or that one element can be a separate structure bonded to or placed with or placed near another element.
"Fiber" refers to a continuous or discontinuous member having a high ratio of length to diameter or width. Thus, a fiber may be a filament, a thread, a strand, a yam, or any other member or combination of these members.
"Layer" when used in the singular can have the dual meaning of a single element or a plurality of elements.
"Liquid impermeable," when used in describing a layer or multi-layer laminate means that liquid, such as urine, will not pass through the layer or laminate, under ordinary use conditions, in a direction generally perpendicular to the plane of the layer or laminate at the point of liquid contact.
"Liquid permeable" refers to any material that is not liquid impermeable.
"Meltblown" refers to fibers formed by extruding a molten thermoplastic material through a plurality of fine, usually circular, die capillaries as molten threads or filaments into converging high velocity gas (e.g., air) streams, generally heated, which attenuate the filaments of molten thermoplastic material to reduce their diameters. Thereafter, the meltblown fibers are carried by the high velocity gas stream and are deposited on a collecting surface to form a web of randomly dispersed meltblown fibers. Such a process is disclosed, for example, in U.S. Patent 3,849,241 to Butin et al. Meltblowing processes can be used to make fibers of various dimensions, including macrofibers (with average diameters from about 40 to about 100 microns), textile-type fibers (with average diameters between about 10 and 40 microns), and microfibers (with average diameters less than about 10 microns). Meltblowing processes are particularly suited to making microfibers, including ultra-fine microfibers (with an average diameter of about 3 microns or less). A description of an exemplary process of making ultra-fine microfibers may be found in, for example, U.S. Patent No. 5,213,881 to Timmons, et al. Meltblown fibers may be continuous or discontinuous and are generally self bonding when deposited onto a collecting surface.
"Member" when used in the singular can have the dual meaning of a single element or a plurality of elements.
"Nonwoven" and "nonwoven web" refer to materials and webs of material that are formed without the aid of a textile weaving or knitting process. For example, nonwoven materials, fabrics or webs have been formed from many processes such as, for example, meltblowing processes, spunbonding processes, air laying processes, and bonded carded web processes.

These terms may be defined with additional language elsewhere in the specification.

It should be noted that a number of the features in this invention implicate a measurement of length. In many cases a simple rule may be used to measure the distance from one edge to another. Of course more sophisticated techniques may be used (e.g., when measuring the perimeter of the leg opening, which can comprise a series of curves). For example, photomicrographs can be prepared and analyzed. Such images could be digitized and appropriate algorithms used to measure length. Alternatively, mechanical devices may be used. Because many of the inventive versions are directed to arrays comprising two products, it is important that the same measurement technique be used when comparing features (e.g., anterior length) of two products in an array.

Generally the gender-specific, disposable, adult-incontinence articles of the array of the present invention are made using methods and processes known in the art, but configured to produce one or more features in the article adapted to be worn by women that are different than one or more features in the article adapted to be worn by men. As noted above, analysis of the physiological characteristics of men and women resulted in gender-specific designs, the features of which are described in more detail in the following paragraphs.

Figure 1 depicts an exemplary version of a disposable, adult-incontinence article adapted to be worn by a man. The figure gives a birds-eye view of a substantially flat, laid-open article before attachment of the side edges of the front portion to the side edges of the back portion to form an article having a waist opening and two leg openings. The front portion, back portion, and their respective side edges are identified below.

As noted above, the article includes a front portion 2 and a back portion 4. The front portion and back portion are interconnected by a crotch portion 6. Generally the term "chassis" is used to refer to the combination comprising a front portion, a back portion, and a crotch portion connecting said front portion to said back portion. The front portion has two side edges 8 and the back portion has two side edges 10. When assembling the article, the side edges 8 are attached to the side edges 10 to form a shape similar to that of underwear and having a waist opening and two leg openings. If desired, the side edges may be releasably engaged to one another (e.g., by using mechanical fasteners such as hook-and-loop-type materials; adhesives; etc.). Also, the side edges of the front and back portions need not be attached directly to one another, but may be attached to one or more intermediate elements (e.g., side panels-not shown in the figures).

The front portion also has a waist-opening front edge 12, and the back portion has a waist-opening back edge 14. When the side edges are joined to one another the waist-opening edges are connected to form a perimeter that typically fits around the lower torso of a user.

For purposes of this application, the edges defining the leg opening are categorized as including a leg-opening front edge 16, a leg-opening back edge 18, and a leg-opening side edge 20. Typically a major portion of the leg-opening side edge runs somewhat parallel to an imaginary longitudinal centerline 22 running from the back to the front of the article. Major portions of the leg-opening front and back edges will typically not run parallel to the longitudinal center line. The leg-opening is categorized in this way to help distinguish certain features of gender-specific articles from one another, as discussed below.

The article will typically include an absorbent core 24. In the depicted representative example, the absorbent core includes fluff (not shown), super absorbent material (not shown), and an optional surge material 26 adapted to help rapidly take in and distribute urine for absorption by the fluff / superabsorbent material. The absorbent core includes an absorbent front edge 28, and absorbent side edges 30. For those articles made by attaching an absorbent assembly or absorbent insert to, for example, an hourglass-like substrate, with the assembly comprising an absorbent core sandwiched between a liquid-permeable, body-side liner, and a liquid-impermeable backing, the liner, backing, or both may extend beyond the edge of the absorbent core itself. For example, in some articles, the liner and backing (i.e., the backsheet of the absorbent core insert) are attached to one another around the perimeter of the absorbent core contained therein, and the area of attachment of these materials may form a flexible flange extending beyond the edge of the absorbent core. Such a flange is not depicted in Figure 1 or Figure 2.

These various edges help further define one or more distances that can vary between a disposable, adult-incontinence article adapted to be worn by a man, and a disposable, adult-incontinence article adapted to be worn by a woman. As noted earlier, the anterior length 32 is the shortest distance between the leg-opening front edge 16 and the waist-opening front edge 12. It should be noted that for purposes of clearly depicting the anterior length in Figure 1, the two-headed arrow representing this distance is slightly offset from one of the side edges 8 of the front portion. If, however, this side edge corresponded to the shortest distance between the leg-opening front edge 16 and the waist-opening front edge 12, then the anterior length 32 would actually overlay the side edge 8.

The waist / absorbent distance 36 is the shortest distance between the absorbent core front edge 28 and the waist-opening front edge 12.

The gasket width 38 is the distance, in the crotch portion, along a line bisecting the middle of the leg-opening side edges and in a transverse direction, between the absorbent core side edge 30 and the outer boundary of the elastic member closest to leg-opening side edge 20. It should be noted that in Figure 1, the two-headed arrow representing this distance touches the leg-opening side edge 20 because of the difficulty in representing the small distances typically characterizing the length between the outer boundary of the elastic member closest to the side edge, and the side edge itself. Of course, in reality, if the outer boundary of the elastic member closest to the leg-opening side edge is inward of the side edge, as is likely the case, then gasket width is measured to the outer boundary of the elastic member-not to the side edge itself.

The crotch width 40 is defined as the shortest distance between the outside boundaries of the opposing, elastic members closest to the opposing leg-opening side edges 20. It should be noted that in Figure 1, the two-headed arrow representing this distance touches the leg-opening side edges 20 because of the difficulty in representing the small distances typically characterizing the length between the outer boundary of the elastic member closest to the side edge, and the side edge itself.

The frontal area of the absorbent core 24 corresponds to the body-facing surface area of that region of the absorbent core between an imaginary transverse line approximately bisecting the article when in a substantially flat, laid-open position (this imaginary line is not shown in Figures 1 and 2, but would approximately overlay the double-headed arrow designated as "40") and the waist-opening front edge.

The frontal transverse span or distance 46 corresponds to the longest distance, in a transverse direction, between the opposing, longitudinal edges 30 of the absorbent core, for that portion of the absorbent core forward of a location between an imaginary transverse line bisecting the article in a substantially flat, laid-open position and the waist-opening front edge (the location of which, for the depicted, representative embodiment, is discussed in the previous paragraph). It should also be noted that, for the depicted embodiment, the side edges 30 of the absorbent core establish a somewhat complex boundary, with a curvilinear portion in the crotch region, and a linear portion in the front region, with each linear portion extending in a substantially perpendicular fashion toward the front edge of the absorbent core.

Figure 2 depicts these same features, but for one representative example of a disposable, adult-incontinence article adapted for use by women. The same numbers used in Figure 1 are used in Figure 2, with the exception that the numbers in Figure 2 are appended with the letter "F" to signify that these features correspond to those employed in an article adapted to be worn by women.

Table 1 below gives a comparison of two articles in a gender-specific array of articles. As noted above in the Summary section, it is not necessary that gender-specific articles in accordance with the present invention incorporate all of the features identified in Table 1. Nor does the invention encompass only those features listed in this table.

**Table 1**

| **Feature** | **Article for Women** | **Article for Men** |
|---|---|---|
| Anterior Length | Shorter | Longer |
| Shape of Leg-Opening Front Edge | More convex toward waist-opening front edge | Less convex toward waist-opening front edge |
| Shape of Leg-Opening Back Edge | More concave toward waist-opening back edge | Less concave toward waist-opening back edge |
| Leg-Opening Perimeter | More | Less |
| Crotch Width | Narrower (Shorter) | Wider (Longer) |
| Waist/Absorbent Distance | Longer | Shorter |
| Frontal Area of Absorbent Core | Less | More |
| Frontal Transverse Span | Shorter | Longer |
| Graphic Disposed on Article | Different | Different |
| Gasket Width | Narrower (Shorter) | Wider (Lonqer) |

As noted elsewhere, one feature of an array of gender-specific articles of the present invention- comprising a disposable, adult-incontinence article adapted to be worn by a woman, and a disposable, adult-incontinence article adapted to be worn by a man-is that the article adapted to be worn by women has a shorter anterior length than the article adapted to be worn by men. This may be achieved, for example, when the leg-opening front edge of the women's article defines a boundary that is more convex toward the waist-opening front edge compared to the boundary defined by the leg-opening front edge of the man's article. It is believed that such convexity helps define a leg opening better suited to the shape of an average female user's thigh compared to the shape of an average male user's thigh. As noted elsewhere, as men age their legs tend to lose muscle mass, which results in thinner legs and a smaller circumference around the upper thigh. Surprisingly, the circumference of the legs of many male users of adult-incontinence products may actually be smaller than the circumference of the legs of many female users of adult-incontinence products. Also, a leg-opening front edge having a shorter anterior length is more likely to result in a boundary that better conforms to the curvature of the upper thigh of the typical female user of the article.

Another feature that may be employed in an array of gender-specific articles is a disposable, adult-incontinence article adapted to be worn by women that comprises a leg-opening back edge that defines a boundary that is more concave in a direction toward the waist-opening back edge compared to the boundary defined by the leg-opening back edge of a corresponding article adapted to be worn by men. A more concave boundary in the woman's article helps ensure coverage of the typical female user's buttocks.

Yet another characteristic that may be selected for use in an array of gender-specific articles is a disposable, adult-incontinence article adapted to be worn by women having a leg-opening perimeter that is greater than the leg-opening perimeter for the corresponding article adapted to be worn by men.

Because of differences in their respective physiologies, an array of gender-specific articles may include a disposable, adult-incontinence article adapted to be worn by women that has a smaller crotch width compared to the corresponding, article for men.

The point at which urine exiting the body contacts a disposable absorbent article is different for men and women, given their physiological differences. Accordingly, the position of the absorbent in an article adapted to be worn by men may be shifted forward in the article, resulting in the distance between the waist-opening front edge and the front edge of the absorbent decreasing. This helps ensure that the point at which urine first contacts the article is on the absorbent core. A women's article will tend to employ the absorbent core at a position ensuring contact with a urination point that is not as far forward as that for a man.

The frontal area and/or frontal transverse span of the absorbent core may also be varied between an article adapted to be worn by men and an article adapted to be worn by women. Because a man's point of urination may move, the overall frontal area of the absorbent core in a disposable, adult-incontinence article adapted to be worn by men may be greater than the corresponding frontal area in a woman's article. Similarly, the frontal transverse span of the absorbent core in a disposable, adult-incontinence article adapted to be worn by men may be greater than the corresponding frontal transverse span of the absorbent core in a woman's article.

As discussed elsewhere, graphics may be disposed on an article adapted to be worn by women that are different than those disposed on an article adapted to be worn by men. In one example, one or more stripes of a desired width are disposed around the perimeter of the article in a location proximate to the waist-opening front edge, back edge, or both. The shape, color, number, or other characteristic of the stripes disposed on a man's article may be different from those disposed on the woman's article. For example, blue-colored stripes may be disposed around the perimeter of the waist region (i.e., that portion of the article proximate to the waist opening) of a man's article, while pink-colored stripes are disposed around the perimeter of the waist region of the corresponding woman's article. In another representative example, graphics may be disposed on the article so that the article more closely resembles the shape and look of woven underwear. So, for example, a graphic fly or opening may be employed on a man's article so that the article resembles men's woven underwear briefs.

Also, the distance between the edge of the absorbent core and the outermost elastic strand proximate to the nearest leg-opening side edge on a man's disposable adult-incontinence article may be greater than that in the corresponding article for a woman. Due to physiological differences between a man and a woman in the area of the crotch, this additional distance generally corresponds to there being additional substrate between the edge of the absorbent core and the outermost elastic member proximate to the side edge of the nearest leg opening. This substrate, which will typically be flexible and incorporate an elastic member proximate to the leg-opening side edge, acts, in a sense, as a gasket to help contain urine. As mentioned elsewhere, the circumference of an older man's leg tends to be smaller due to the loss of muscle mass, and often may be less than that of a woman. Accordingly, an increased gasket width helps to ensure the presence of sufficient material between the inner surface of a man's thigh and the absorbent core.

The preceding paragraphs outline various features that may be employed in an array of gender-specific, disposable, adult-incontinence articles, with these features differing in some way between an article adapted to be worn by a woman and an article adapted to be worn by a man. The article itself may employ many different components and subassemblies, so long as the array of gender-specific articles employs one or more of the novel features discussed elsewhere in this application. For example, the article may comprise an hour-glass-shaped liquid-impermeable substrate, such as a film, attached to an hour-glass-shaped liquid-permeable substrate, such as a nonwoven, with a cellulosic-fluff / superabsorbent-material absorbent core disposed between the liquid-impermeable and liquid-permeable substrate. Either of these substrates can be more complicated of course (e.g., laminates of nonwovens, films, or both; co-form materials; etc.), as can the absorbent core. Also, various pigments, odor-control agents, fillers, and other materials may be employed. Elastic materials such as strands or webs may be employed to help give the article elastomeric qualities in desired locations. For example, elastic members such as strands are often employed in the vicinity of the leg openings and waist to help ensure a close fit. Also, elastic members such as strands are often positioned below the waist-opening to effect a close fit around the lower torso of a user. For example, spaced-apart, substantially parallel elastic strands may be sandwiched between two nonwoven materials in the vicinity of the torso. Often the strands are attached to nonwoven facings, typically using adhesive, when the strands are in a stretched condition. The resulting laminate-elastic strands sandwiched between two nonwoven facings-is then allowed to retract, producing a gathered substrate capable of stretching and recovering its shape.

Note too that many different components and subassemblies may be used in disposable, adult-incontinence articles. For example, rather than the hourglass-shaped liquid-impermeable and liquid-permeable substrates described above, a single, hourglass-shaped liquid-permeable substrate may be formed (e.g., two polypropylene facings attached to one another, with elastic strand disposed between the facings in certain locations, such as proximate to the leg openings, waist opening, and those portions of the front and back of the article that will contact the lower torso of a user). To this liquid-permeable substrate or chassis is attached an absorbent core or assembly, with the core or assembly comprising a cellulosic-fluff/superabsorbent material sandwiched between a liquid-impermeable backing, such as a film, and a liquid-permeable liner, such as a nonwoven. When the absorbent assembly is attached to the hourglass-shaped, liquid-permeable, chassis, the liquid-impermeable backing is attached to the chassis.

Other configurations are possible. For example, the article may be made by forming a front panel (or portion), a back panel (or portion), and attaching the two with a crotch portion comprising an absorbent assembly. And such articles may employ containment flaps, various fastening systems (including, e.g., refastenable systems such as those using hook-and-loop-type fasteners), separate waistbands attached to the article, side panels (e.g., elastomeric side panels), and many other such components or assemblies. It should be readily understood that the present invention encompasses many such configurations of disposable articles, so long as said articles employ one or more of the identified inventive features which are varied between an article adapted to be worn by women and an article adapted to be worn by men Similarly, many different methods, unit operations, and processes may be used when making gender-specific, disposable, adult-incontinence articles.

Of course the articles themselves are typically stored, shipped, and sold in packages, such as bags made of film

### Examples

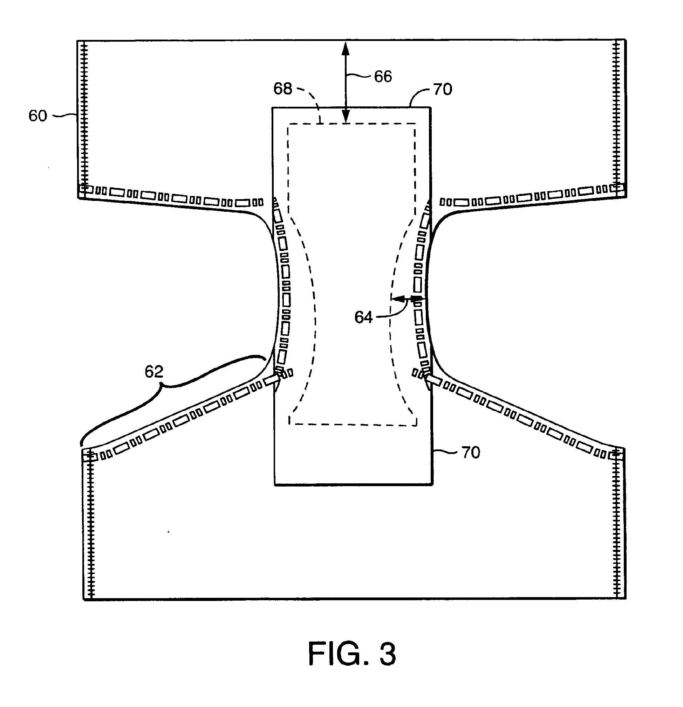

The following example representatively illustrates one embodiment of the present invention. An array of gender-specific, disposable adult-incontinence articles was made. The articles were made using conventional materials and processes, and generally corresponded to the shapes depicted in Figures 1 and 2. More detailed drawings generally corresponding to those articles that were made are presented in Figures 3 (male article) and 4 (female article).

The materials employed included polypropylene nonwoven materials for much of the chassis construction; film as the moisture barrier / backsheet / backing for the absorbent assembly; an absorbent core comprising cellulosic fluff and superabsorbent material; and elastic strand around the waist opening, leg opening, and portions of the front and back panels / portions of the article. Hot-melt adhesive or ultrasonic energy was used to attach the components to one another. In this exemplary embodiment, a polypropylene nonwoven material having an hourglass-like shape served as the outer cover. To this were attached nonwoven front and back panels at the front and back portions of the article (with elastic strand sandwiched at various locations between these nonwoven front and back panels and the nonwoven outer cover). The front panel extended longitudinally from a location proximate to the waist-opening front edge to a location proximate to the crotch portion (but did not substantially overlay the crotch portion of the hourglass-like outer cover). The back panel extended longitudinally from a location proximate to the waist-opening back edge to a location proximate to the crotch portion (but, like the front panel, did not substantially overlay the crotch portion of the hourglass-like polypropylene outercover). Both the front panel and the back panel extended transversely from a location proximate to one side edge to the opposing side edge of the front portion and the back portion of the article.

An absorbent insert comprising a fluff / superabsorbent core sandwiched between a barrier/backsheet material (a film) and a body-side liner (a nonwoven material) was attached to the aforementioned assembly of an hourglass-like shaped outer cover having elastic strand sandwiched between front and back nonwoven panels at the front portion and the back portion of the article. Approximate dimensions and inter-relationships between the two articles are given in Table 2.

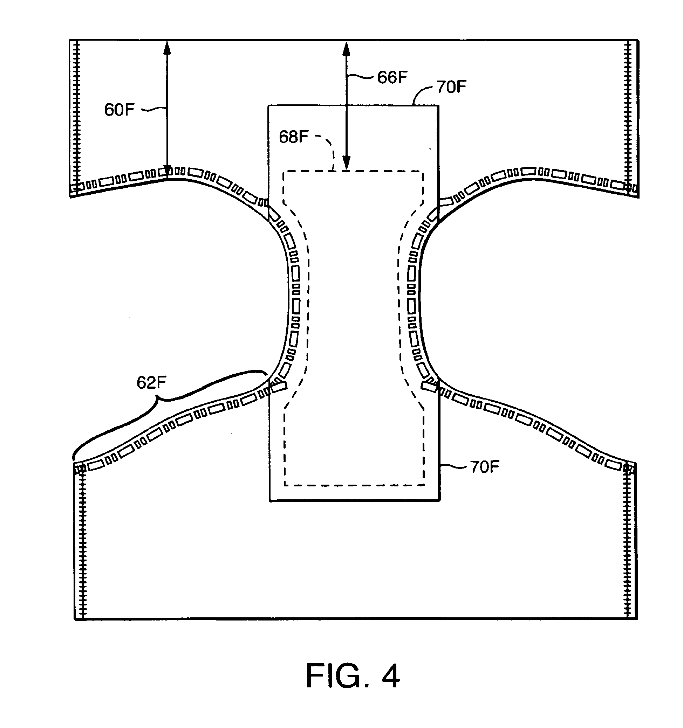

The anterior length 60 for the men's article was about 210 millimeters, and overlaid the side edge of the front portion, given that the leg-opening front edges were generally linear, and proceeded upward at a slight angle from the a location proximate to the crotch portion to the side edge itself (see Figure 3). The anterior length 60F for the women's article was about 190 millimeters, however, and was measured from the point on the leg-opening front edge closest to the waist-opening front edge (see Figure 4). This line, which is parallel to the longitudinal centerline of the article, is inward of the side edge of the front portion, given that the curvilinear boundary established by the leg-opening front edge of the women's article is more convex in a direction toward the waist opening front edge, compared to the corresponding, linear boundary established by the leg-opening front edge of the corresponding men's article. The leg-opening front edge of the women's article, having greater convexity, is better adapted to conform to the curved surface of the upper thigh region of a typical female user of such disposable, adult-incontinence articles.

An examination of Figure 3 shows that the leg-opening back edges 62 of this exemplary version of an article adapted to be worn by men was also substantially linear, and proceeded downward at some angle from a location proximate to the crotch portion to the side edge of the back portion. The leg-opening back edge 62F of the exemplary women's article, however, does not define a substantially linear boundary, and instead defines a curvilinear boundary that is more concave in a direction toward the waist-opening back edge. The leg-opening back edge of the women's article, having greater concavity, is better adapted to ensure coverage of the buttocks region of a typical female user of such disposable, adult-incontinence articles.

Table 2 also shows that the perimeter of the leg-opening (which equates to the sum of the lengths of the leg-opening back, side, and front edges) for the women's article is greater than the perimeter of the leg-opening of the men's article. Again, this leg-opening feature is consistent with our observation that men, as they age, tend to lose muscle mass in their legs, resulting in many male adult-incontinence-product users typically having a smaller thigh circumference than many female adult-incontinence-product users.

Furthermore, Table 2 shows that the man's article has a crotch width and a gasket width greater than the crotch width and gasket width of the corresponding women's article. The gasket width 64 (see Figure 3) of the men's article was about 40 millimeters and the gasket width of the women's article was about 20 millimeters (not shown on Figure 4 due to the close proximity of the edge of the absorbent core to the outermost elastic member nearest the leg-opening side edge, but measured at the same location as the corresponding location on the men's article: along a line in a transverse direction intersecting the center of the leg-opening side edge).

Also, the location of the absorbent was shifted forward for the men's article, compared to the women's article, to accommodate the point at which urine typically exits a man's body compared to a women's body. Accordingly, the waist/absorbent distance 66 for the men's article was about 120 millimeters, and the waist/absorbent distance for the corresponding women's article 66F was about 180 millimeters. It should be noted that these distances were determined from the edge of the absorbent core (68 and 68F, respectively, in Figures 3 and 4). The edges 70 and 70F corresponds to the edge of the substrates encasing the absorbent core, with these substrates extending beyond the absorbent core itself to create a flexible flange surrounding the core. As discussed above, gasket width and waist/absorbent length / distance is measured from the edge of the absorbent core, not the edge of any substrate encasing and extending substantially beyond the edge of the absorbent core. It should also be noted that Figures 1 and 2 show the absorbent core, and a surge material, but not the outer perimeter of any substrate encasing the absorbent core and surge material. Figures 3 and 4, on the other hand, do not show a surge material, but do show both the absorbent core and the perimeter of the substrates used to encase the absorbent core.

Finally, while not shown, different colored stripes were disposed around each article at a location proximate to the waist opening. In this representative embodiment, a plurality of blue stripes were disposed around the perimeter of the men's article, and a plurality of pink stripes were disposed around the perimeter of the women's article.

**Table 2**

| **Feature** | **Article for Women** | **Article for Men** |
|---|---|---|
| Anterior Length | 190 mm | 210 mm |
| Shape of Leg-Opening Front Edge | More convex toward waist-opening front edge | Less convex toward waist-opening front edge |
| Shape of Leg-Opening Back Edge | More concave toward waist-opening back edge | Less concave toward waist-opening back edge |
| Leg-Opening Perimeter | 785 mm | 740 mm |
| Crotch Width | 155 mm | 195 mm |
| Waist/Absorbent Distance | 180 mm | 120 mm |
| Frontal Area of Absorbent Core | Less | More |
| Graphic Disposed on Article | Pink-colored stripes proximate to waist opening | Blue-colored stripes proximate to waist opening |
| Gasket Width | 20 mm | 40 mm |

## Claims

1. An array of gender-specfic, disposable, adult-incontinence articles, the array comprising:
a first disposable, adult-incontinence article adapted to be worn by women, the article comprising:
a chassis comprising a front portion (2F), a back portion (4F), and a crotch portion (6F) connecting said front and back portions (2F, 4F); wherein said chassis has a leg-opening front edge (16F) establishing a boundary having a first convexity, a leg-opening side edge (20F), a leg-opening back edge (18F) establishing a first concavity; a waist-opening front edge (12F), and a first crotch width (40F);
an absorbent core (14F) having a front edge (28F), a side edge (30F), a first frontal area, and a first frontal transverse span (46F), wherein the absorbent core (24F) is attached to said chassis, wherein said first article has a first anterior length (32F) equaling the shortest distance between the leg-opening front edge (16F) and the waist opening front edge (12F); a first gasket width (38F); and a first waist / absorbent distance (36F) equaling the shortest distance between the front edge (28F) of the absorbent core (24F) and the walst-opening front edge (12F); and
a second disposable, adult-incontinence article adapted to be worn by men, the article comprising:
a chassis comprising a front portion (2), a back portion (4), and a crotch portion (6) joining said front and back portions (2, 4); wherein said chassis has a leg-opening front edge (16) establishing a boundary having a second convexity, a leg-opening side edge (16), a leg-opening back edge (18) establishing a boundary having a second concavity; a waist-opening front edge (12), a waist-opening back edge (14), and a second crotch width (40);
an absorbent core (24) having a front edge (28), a side edge (30), a second frontal area, and a second frontal transverse span (46), wherein the absorbent core (24) is attached to said chassis, wherein said second article has a second anterior length (32) equaling the shortest distance between the leg-opening front edge (16) and the waist opening front edge (12); a second gasket width (38); and a second waist / absorbent distance (36) equaling the shortest distance between the front edge (28) of the absorbent core (24F) and the front edge (12) of the waist opening, wherein the difference between the first article and the second article is selected from the group consisting of: the first anterior length (32F) being less than the second anterior length (32), the first gasket width (38F) being less than the second gasket width (38), the first waist / absorbent distance (36f) being more than the second waist / absorbent distance (36), the first convexity being greater than the second convexity, the first concavity being greater than the second concavity, the first frontal area being less than the second frontal area, the first frontal transverse span (46F) being less than the second frontal transverse span (46), or some combination thereof.

## Patentansprüche

1. Anordnung aus geschlechtsspezifischen Einweg-Inkontinenzartikeln für Erwachsene, wobei die Anordnung umfasst:
einen ersten Einweg-Inkontinenzartikel für Erwachsene, der geeignet ist, von Frauen getragen zu werden, wobei der Artikel umfasst:
einen Rahmen, der einen vorderen Teil (2F), einen hinteren Teil (4F) und einen Schrittteil (6F) umfasst, welcher den vorderen und hinteren Teil (2F, 4F) verbindet; wobei der Rahmen einen Beinöffnungsvorderrand (16F), welcher eine Grenze mit einer ersten Konvexität bereitstellt, einen Beinöffnungsseitenrand (20F), einen Beinöffnungshinterrand (18F), welcher eine erste Konkavität bereitstellt, einen Taillenöffnungsvorderrand (12F) und eine erste Schrittbreite (40F) aufweist;
einen absorptionsfähigen Kern (14F), welcher einen Vorderrand (28F), einen Seitenrand (30F), einen ersten Frontalbereich und eine erste frontale Querspanne (46F) aufweist, wobei der absorptionsfähige Kern (24F) an dem Rahmen befestigt ist, wobei der erste Artikel eine erste anteriore Länge (32F), die der kürzesten Distanz zwischen dem Beinöffnungsvorderrand (16F) und dem Taillenöffnungsvorderrand (12F) gleicht, eine erste Dichtungsbreite (38F); und eine erste Taillen/ Absorptionsmitteldistanz (36F) aufweist, die der kürzesten Distanz zwischen dem Vorderrand (28F) des absorptionsfähigen Kerns (24F) und dem Taillenöffnungsvorderrand (12F) gleicht; und
einen zweiten Einweg-Inkontinenzartikel für Erwachsene, der geeignet ist, von Männern getragen zu werden, wobei der Artikel umfasst:
einen Rahmen, der einen vorderen Teil (2), einen hinteren Teil (4) und einen Schrittteil (6) umfasst, welcher den vorderen und hinteren Teil (2, 4) verbindet; wobei der Rahmen einen Beinöffnungsvorderrand (16), welcher eine Grenze mit einer zweiten Konvexität bereitstellt, einen Beinöffnungsseitenrand (16), einen Beinöffnungshinterrand (18), welcher eine Grenze mit einer zweiten Konkavität bereitstellt, einen Taillenöffnungsvorderrand (12), einen Taillenöffnungshinterrand (14) und eine zweite Schrittbreite (40) aufweist;
einen absorptionsfähigen Kern (24), welcher einen Vorderrand (28), einen Seitenrand (30), einen zweiten Frontalbereich und eine zweite frontale Querspanne (46) aufweist, wobei der absorptionsfähige Kern (24) an dem Rahmen befestigt ist, wobei der zweite Artikel eine zweite anteriore Länge (32), die der kürzesten Distanz zwischen dem Beinöffnungsvorderrand (16) und dem Taillenöffnungsvorderrand (12) gleicht; eine zweite Dichtungsbreite (38); und eine zweite Taillen/ Absorptionsmitteldistanz (36) aufweist, die der kürzesten Distanz zwischen dem Vorderrand (28) des absorptionsfähigen Kerns (24F) und dem Vorderrand (12) der Taillenöffnung gleicht, wobei der Unterscheid zwischen dem ersten Artikel und dem zweiten Artikel ausgewählt ist aus der Gruppe bestehend aus: die erste anteriore Länge (32F) ist geringer als die zweite anteriore Länge (32), die erste Dichtungsbreite (38F) ist geringer als die zweite Dichtungsbreite (38), die erste Taillen/Absorptionsmitteldistanz (36F) ist größer als die zweite Taillen/Absorptionsmitteldistanz (36), die erste Konvexität ist größer als die zweite Konvexität, die erste Konkavität ist größer als die zweite Konkavität, der erste Frontalbereich ist geringer als der zweite Frontalbereich, die erste frontale Querspanne (46F) ist geringer als die zweite frontale Querspanne (46), oder eine Kombination davon.

## Revendications

1. Ensemble d'articles jetables et spécifiques au sexe pour incontinents adultes, ledit ensemble comprenant :
un premier article jetable pour incontinents adultes, adapté pour être porté par des femmes, l'article comprenant :
une structure comprenant une partie avant (2F), une partie arrière (4F) et une partie d'entrejambe (6F) reliant lesdites parties avant et arrière (2F, 4F) ; ladite structure ayant un bord avant d'ouverture de jambe (16F) réalisant une bordure ayant une première convexité, un bord latéral d'ouverture de jambe (20F), un bord arrière d'ouverture de jambe (18F) établissant une première concavité ; un bord avant d'ouverture de taille (12F) ; et une première largeur d'entrejambe (40F) ;
un coeur absorbant (24F) ayant un bord avant (28F), un bord latéral (30F), une première zone frontale et un premier pan transversal frontal (46F), le coeur absorbant (24F) étant attaché à ladite structure, ledit premier article ayant une première longueur antérieure (32F) égale à la distance la plus courte entre le bord avant d'ouverture de jambe (16F) et le bord avant d'ouverture de taille (12F) ; une première largeur de joint (38F) ; et une première distance (36F) entre la taille et le coeur absorbant, égale à la distance la plus courte entre le bord avant (28F) du coeur absorbant (24F) et le bord avant d'ouverture de taille (12F) ; et
un deuxième article jetable pour incontinents adultes, adapté pour être porté par des hommes, l'article comprenant :
une structure comprenant une partie avant (2), une partie arrière (4) et une partie d'entrejambe (6) reliant lesdites parties avant et arrière (2, 4) ; ladite structure ayant un bord avant d'ouverture de jambe (16) réalisant une bordure ayant une deuxième convexité, un bord latéral d'ouverture de jambe (20), un bord arrière d'ouverture de jambe (18) établissant une deuxième concavité ; un bord avant d'ouverture de taille (12) ; un bord arrière d'ouverture de taille (14) et une deuxième largeur d'entrejambe (40) ;
un coeur absorbant (24) ayant un bord avant (28), un bord latéral (30), une deuxième zone frontale et un deuxième pan transversal frontal (46), le coeur absorbant (24) étant attaché à ladite structure, ledit deuxième article ayant une deuxième longueur antérieure (32) égale à la distance la plus courte entre le bord avant d'ouverture de jambe (16) et le bord avant d'ouverture de taille (12) ; une deuxième largeur de joint (38) ; et une deuxième distance (36) entre la taille et le coeur absorbant, égale à la distance la plus courte entre le bord avant (28) du coeur absorbant (24F) et le bord avant d'ouverture de taille (12) ; la différence entre le premier article et le deuxième article étant sélectionnée parmi le groupe constitué des caractéristiques suivantes : la première longueur antérieure (32F) est inférieure à la deuxième longueur antérieure (32), la première largeur de joint (38F) est inférieure à la deuxième largeur de joint (38), la première distance (36f) entre la taille et le coeur absorbant est supérieure à la deuxième distance (36) entre la taille et le coeur absorbant, la première convexité est supérieure à la deuxième convexité, la première concavité est supérieure à la deuxième concavité, la première zone frontale est inférieure à la deuxième zone frontale, le premier pan transversal frontal (46F) est inférieur au deuxième pan transversal frontal (46), ou une combinaison de celles-ci.
